# EUROPEAN PATENT APPLICATION

(11) **EP 4 385 510 A1**
(43) Date of publication of application: **19.06.2024**
(21) Application number: 22855304.6
(22) Date of filing: 04.08.2022
(51) Int. Cl.: A61K 31/501, A61P 35/00

(54) **USE OF ENSARTINIB OR SALT THEREOF IN TREATMENT OF DISEASE CARRYING MET 14 EXON SKIPPING MUTATION**

(30) Priority: 10.08.2021 WO PCT/CN2021/111878
(71) Applicant: Betta Pharmaceuticals Co., Ltd, Yuhang Hangzhou Zhejiang 311100 (CN)
(72) Inventor: LIU, Lijia, Beijing 100176 (CN); GUO, Jing, Beijing 100176 (CN); WANG, Yu, Beijing 100176 (CN); ZHAO, Xiangdong, Beijing 100176 (CN); CHEN, Jie, Beijing 100176 (CN); WANG, Yang, Beijing 100176 (CN); YUAN, Xiaobin, Beijing 100176 (CN); JI, Dong, Beijing 100176 (CN); KONG, Licheng, Hangzhou, Zhejiang 311100 (CN); DING, Lieming, Hangzhou, Zhejiang 311100 (CN)
(74) Representative: IPAZ
(86) International application number: PCT/CN2022/110160
(87) International publication number: WO 2023/016321

(57) **Abstract**

Embodiments of the present application provide Ensartinib or a salt thereof and a use of a composition containing Ensartinib or the salt thereof in treatment of a disease carrying MET 14 exon skipping mutation.

## Description

This application claims priority to and benefit of PCT application No. PCT/CN2021/111878, filed on August 10, 2021, submitted to the International Bureau, and the invention name is "Use of ensartinib or its salts in the treatment of diseases caused by MET14 exon skipping mutations", the disclosures of which are hereby incorporated herein by reference.

### FIELD OF THE APPLICATION

This application relates to the technical field of new uses of ensartinib, and in particular relates to the use of ensartinib or salt thereof in treatment of diseases carrying MET 14 exon skipping mutation.

### BACKGROUND

The MET gene is located on chromosome 7 band 7q21-q31, includes 21 exons. c-MET is a transmembrane receptor tyrosine kinase encoded by the MET gene and belongs to receptor tyrosine kinase (RTK) family, it is associated with a variety of oncogene products and regulatory proteins, plays essential roles in cell proliferation, differentiation and motility. Abnormal activation of c-Met pathway includes MET exon 14 skipping mutation, MET amplification and c-MET protein overexpression. (Organ S L, Tsao M S. An overview of the c-MET signaling pathway[J]. Therapeutic Advances in Medical Oncology, 2011, 3(1 Suppl).).

MET exon 14 encodes 141 amino acids, it is located in juxtamembrane (JM) domain which is a negative regulatory region of c-Met activity, including the binding site of E3 ubiquitin ligase and c-Cbl tyrosine binding site (Y1003), it involves in the ubiquitination and degradation of c-MET protein.

The MET exon 14 skipping mutation primarily occurs due to the splicing process after transcription of the MET gene, where the 14th exon is spliced together with the flanking introns, resulting in mature mRNA with 141 fewer base pairs than normal splicing. This process generates mature mRNA producing the MET exon 14, subsequently translated by ribosomes into a c-Met receptor protein lacking 47 amino acids (L964-D1010) (Pilotto S, Gkountakos A, Carbognin L, et al. MET exon 14 juxtamembrane splicing mutations: Clinical and therapeutical perspectives for cancer therapy[J]. Annals of Translational Medicine, 2017, 5(1):2-2). This mutation leads to the deletion of the juxtamembrane (JM) domain containing the E3 ubiquitin ligase c-Cbl binding site, preventing the binding of c-Cbl to the c-Met protein. Consequently, ubiquitination and degradation of c-MET protein are restricted, resulting in increased stability of c-MET and continuous activation of downstream signals of MET, ultimately becoming one of the driving genes in cancer.

In cancer treatment, targeted drugs against specific oncogenic drivers play a crucial role. For instance, in non-small cell lung cancer (NSCLC), known drugs for treating NSCLC include osimertinib for the EGFR T790M mutation and TAK-788 by Takeda for EGFR exon 20 insertion mutations. Different targeted drugs show promising therapeutic effects against cancers caused by specific mutations, but the efficacy against cancers caused by other gene mutations remains unpredictable. MET exon 14 skipping mutations occur in 3-4% of newly diagnosed advanced NSCLC cases, associated with poor prognosis. There is a need to develop more drugs targeting MET exon 14 skipping mutations in advanced NSCLC and other diseases carrying such mutations.

Ensartinib is an approved drug for treating ALK-positive non-small cell lung cancer (NSCLC). However, its potential effectiveness in treating diseases caused by MET exon 14 skipping mutations, such as advanced NSCLC with MET exon 14 skipping mutations, has not been studied.

### SUMMARY

The inventor of this application unexpectedly discovered during research that ensartinib or salt thereof has the effect of treating diseases carrying MET exon 14 skipping mutation, and this application is completed based on this.

The present application first provides the use of ensartinib or a salt thereof, or a composition comprising ensartinib or a salt thereof, in the treatment of diseases carrying MET exon 14 skipping mutation, wherein, ensartinib has the structure shown in Formula I:

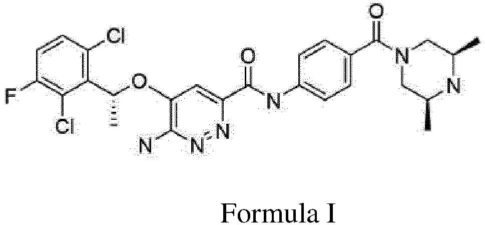

The present application secondly provides the use of ensartinib or a salt thereof, or a composition comprising ensartinib or a salt thereof, in the preparation of a medicament for treating diseases carrying MET exon 14 skipping mutation, wherein, ensartinib has the structure shown in Formula I:

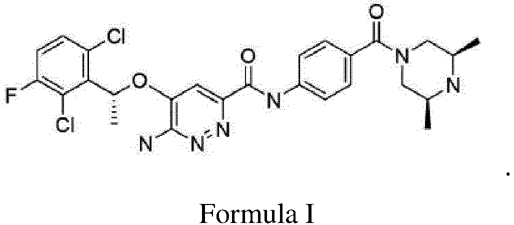

The present application thirdly provides a method of treating diseases carrying MET exon 14 skipping mutation, which involves administering a therapeutically effective amount of ensartinib or a salt thereof, or a composition comprising ensartinib or a salt thereof to a subject in need, wherein, ensartinib has the structure shown in Formula I:

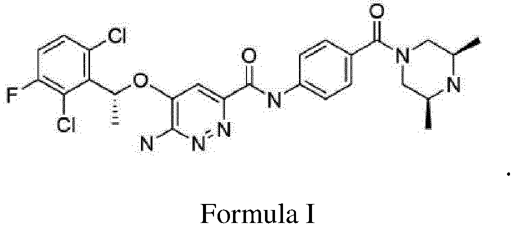

Ensartinib or a salt thereof, or a composition comprising ensartinib or a salt thereof of present application can significantly inhibit MET exon 14 skipping mutation kinase activity, and proliferation of human gastric cancer Hs746T cell carrying MET exon 14 skipping mutation, and shows great anti-tumor activity against xenograft tumor model carrying MET exon 14 skipping mutation in vivo, and shows significant efficacy in clinical tirals. Therefore, ensartinib or a salt thereof, or a composition comprising ensartinib or a salt thereof can be used to treat diseases carrying MET exon 14 skipping mutation, and can further be used to prepare a medicament for treating diseases caused by MET exon 14 skipping mutation.

### DESCRIPTION OF THE DRAWINGS

In order to explain the embodiments of the present application or the technical solutions in the prior art more clearly, the drawings needed to be used in the description of the embodiments or the prior art will be briefly introduced below. Obviously, the drawings in the following description are only one embodiment of the present application, for those of skill in the art, other embodiments can be obtained based on these drawings.
Figure 1 is the relationship curve between MET exon 14 skipping mutation kinase inhibition rate and the concentration of ensartinib.
Figure 2 is the relationship curve between Hs746T cell survival rate and ensartinib concentration curve.
Figure 3 is the tumor growth curve of tumor volume in mice treated with different doses of ensartinib hydrochloride.

### EXAMPLES

In order to make the purpose, technical solutions, and advantages of the present application clearer, the present application will be further described in detail below with reference to the accompanying drawings and examples. Obviously, the described embodiments are only some of the embodiments of the present application, but not all of the embodiments. All other embodiments obtained by those of ordinary skill in the art based on the embodiments in this application fall within the scope of protection of this application.

In the present application, "administration" and "treatment," as it applies to an animal, human, experimental subject, cell, tissue, organ, or biological fluid, refers to contact of an exogenous pharmaceutical, therapeutic, diagnostic agent, or composition to the animal, human, subject, cell, tissue, organ, or biological fluid. "Treat" or "treating", as used herein, means to administer ensartinib or a salt thereof, or a composition comprising ensartinib or a salt thereof, to a subject having a disease carrying MET exon 14 skipping mutation, or diagnosed with a disease carrying MET exon 14 skipping mutation, to achieve at least one positive therapeutic effect, such as for example, when said disease is a cancer, reduced number of cancer cells, reduced tumor size, reduced rate of cancer cell infiltration into peripheral organs, or reduced rate of tumor metastasis or tumor growth. "Treatment" may include: halting or delaying the progression of disease carrying MET exon 14 skipping mutation, stabilization of disease carrying MET exon 14 skipping mutation, ameliorating or abrogating the clinical manifestations of disease carrying MET exon 14 skipping mutation, reducing the severity or duration of the clinical symptoms of disease carrying MET exon 14 skipping mutation, prolonging the survival of a patient relative to the expected survival in a similar untreated patient, and inducing complete or partial remission of a disease carrying MET exon 14 skipping mutation. For example, when said disease is a cancer, "treatment" may include one or more of the following: decreasing the number of one or more tumor markers, halting or delaying the growth of a tumor, inhibiting the growth or survival of tumor cells, eliminating or reducing the size of one or more cancerous lesions or tumors, decreasing the level of one or more tumor markers, inducing complete or partial remission of of a cancerous condition.

As used herein, the term "effective amount" refers to an amount which, when administered in a proper dosing regimen, is sufficient to reduce or ameliorate the severity, duration or progression of the disorder being treated, prevent the advancement of the disorder being treated, cause the regression of the disorder being treated, or enhance or improve the prophylactic or therapeutic effect(s) of another therapy.

As used herein, unless otherwise indicated, the term "subject" refers to animals such as mammals, including, but not limited t o, primates (e.g., humans) , cows, sheep, goats, horses, dogs, cats, rabbits, rats, mic e and the like. In certain embodiments, the subject is a human. The term "subject" or "patient" are used interchangeably herein when referring to, for example, a mammalian subject (eg, a human).

The term "about" refers to variation in the numerical quantity that can occur, for example, through typical measuring, handling and sampling procedures involved in the preparation, characterization and/or use of the substance or composition; through inadvertent error in these procedures; through differences in the manufacture, source, or purity of the ingredients employed to make or use the compositions or carry out the procedures; and the like. In certain embodiments, "about" can mean a variation of ±0 .1%, ± 0 .5%, ± 1%, ± 2%, ± 3%, ± 4%, ± 5%, ± 6%, ± 7%, ± 8%, ± 9% or ± 10%.

The present application first provides the use of ensartinib or a salt thereof, or a composition comprising ensartinib or a salt thereof, in the treatment of diseases carrying MET exon 14 skipping mutation, wherein, ensartinib has the structure shown in Formula I:

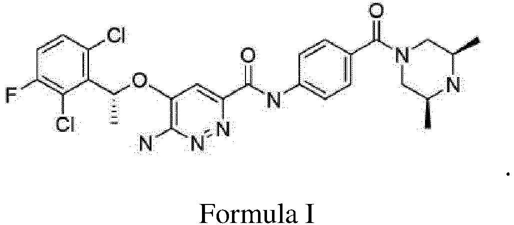

The present application secondly provides the use of ensartinib or a salt thereof, or a composition comprising ensartinib or a salt thereof, in the preparation of a medicament for treating diseases carrying MET exon 14 skipping mutation, wherein, ensartinib has the structure shown in Formula I:

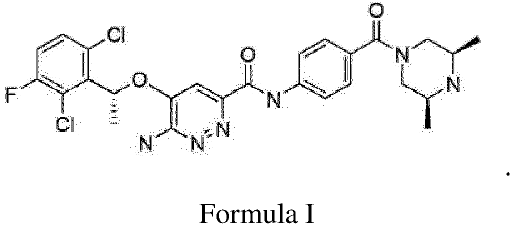

Preferably, the diseases carrying MET exon 14 skipping mutation include at least one of lung cancer, colon cancer, breast cancer, prostate cancer, liver cancer, pancreatic cancer, brain cancer, kidney cancer, ovarian cancer, stomach cancer, skin cancer, bone cancer, glioma, papillary renal cell carcinoma, or head and neck squamous cell carcinoma.

Preferably, the diseases carrying MET exon 14 skipping mutation is non-small cell lung cancer.

The composition described in the present application comprising an effective amount of ensartinib, or if applicable, a pharmaceutically acceptable salt, polymorphic form of a salt, solvate, hydrate, or polymorphic form of the compound, or prodrug; and an acceptable carrier.

"Pharmaceutically acceptable salt", "salt" mentioned in the present application include salts formed by alkali ions and free acids, such as hydrochloride, phosphate, formate, acetate, fumarate, and oxalate, maleate, citrate, etc.

Preferably, a composition of this invention is formulated for pharmaceutical use ("a pharmaceutical composition"), wherein the carrier is a pharmaceutically acceptable carrier. The carrier(s) must be "acceptable" in the sense of being compatible with the other ingredients of the formulation and, in the case of a pharmaceutically acceptable carrier, not deleterious to the recipient thereof in amounts typically used in medicaments.

Pharmaceutically acceptable carriers, adjuvants and vehicles that may be used in the pharmaceutical compositions of this invention include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene- polyoxypropylene-block polymers, polyethylene glycol and wool fat.

The pharmaceutical compositions of the invention include those suitable for oral, rectal, nasal, topical (including buccal and sublingual), vaginal or parenteral (including subcutaneous, intramuscular, intravenous and intradermal) administration. In certain embodiments, ensartinib is administered transdermally (e.g., using a transdermal patch). Other formulations may conveniently be presented in unit dosage form, e.g., tablets and sustained release capsules, and in liposomes, and may be prepared by any methods well known in the art of pharmacy. See, for example, Remington's Pharmaceutical Sciences, Mack Publishing Company, Philadelphia, PA (17th ed. 1985).

Such preparative methods include the step of bringing into association with the molecule to be administered ingredients such as the carrier that constitutes one or more accessory ingredients. In general, the compositions are prepared by uniformly and intimately bringing into association the active ingredients with liquid carriers, liposomes or finely divided solid carriers or both, and then if necessary shaping the product.

In certain preferred embodiments, the compound is administered orally. Compositions of the present invention suitable for oral administration may be presented as discrete units such as capsules, sachets or tablets each containing a predetermined amount of the active ingredient; as a powder or granules; as a solution or a suspension in an aqueous liquid or a non-aqueous liquid; or as an oil-in-water liquid emulsion or a water-in-oil liquid emulsion, or packed in liposomes and as a bolus, etc. Soft gelatin capsules can be useful for containing such suspensions, which may beneficially increase the rate of compound absorption.

A tablet may be made by compression or molding, optionally with one or more accessory ingredients. Compressed tablets may be prepared by compressing in a suitable machine the active ingredient in a free- flowing form such as a powder or granules, optionally mixed with a binder, lubricant, inert diluent, preservative, surface-active or dispersing agent. Molded tablets may be made by molding in a suitable machine a mixture of the powdered compound moistened with an inert liquid diluent. The tablets optionally may be coated or scored and may be formulated so as to provide slow or controlled release of the active ingredient therein. Methods of formulating such slow or controlled release compositions of pharmaceutically active ingredients, such as those herein and other compounds known in the art, are known in the art and described in several issued US Patents, some of which include, but are not limited to, US Patent Nos. 4,369,172; and 4,842,866, and references cited therein. Coatings can be used for delivery of compounds to the intestine (see, e.g., U.S. Patent Nos. 6,638,534, 5,217,720, and 6,569,457, 6,461,631, 6,528,080, 6,800,663, and references cited therein). A useful formulation for the compounds of this invention is the form of enteric pellets of which the enteric layer comprises hydroxyl propyl methyl cellulose acetate succinate.

In the case of tablets for oral use, carriers that are commonly used include lactose and corn starch. Lubricating agents, such as magnesium stearate, are also typically added. For oral administration in a capsule form, useful diluents include lactose and dried cornstarch. When aqueous suspensions are administered orally, the active ingredient is combined with emulsifying and suspending agents. If desired, certain sweetening and/or flavoring and/or coloring agents may be added.

Compositions suitable for topical administration include lozenges comprising the ingredients in a flavored basis, usually sucrose and acacia or tragacanth; and pastilles comprising the active ingredient in an inert basis such as gelatin and glycerin, or sucrose and acacia.

Compositions suitable for parenteral administration include aqueous and nonaqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example, sealed ampules and vials, and may be stored in a freeze dried (lyophilized) condition requiring only the addition of the sterile liquid carrier, for example water for injections, immediately prior to use. Extemporaneous injection solutions and suspensions may be prepared from sterile powders, granules and tablets.

Such injection solutions may be in the form, for example, of a sterile injectable aqueous or oleaginous suspension. This suspension may be formulated according to techniques known in the art using suitable dispersing or wetting agents (such as, for example, Tween 80) and suspending agents. The sterile injectable preparation may also be a sterile injectable solution or suspension in a non-toxic parenterally-acceptable diluent or solvent, for example, as a solution in 1 ,3-butanediol. Among the acceptable vehicles and solvents that may be employed are mannitol, water, Ringer's solution and isotonic sodium chloride solution, hi addition, sterile, fixed oils are conventionally employed as a solvent or suspending medium. For this purpose, any bland fixed oil may be employed including synthetic mono- or diglycerides. Fatty acids, such as oleic acid and its glyceride derivatives are useful in the preparation of injectables, as are natural pharmaceutically- acceptable oils, such as olive oil or castor oil, especially in their polyoxyethylated versions. These oil solutions or suspensions may also contain a long-chain alcohol diluent or dispersant.

The pharmaceutical compositions of this invention may be administered in the form of suppositories for rectal administration. These compositions can be prepared by mixing a compound of this invention with a suitable non-irritating excipient which is solid at room temperature but liquid at the rectal temperature and therefore will melt in the rectum to release the active components. Such materials include, but are not limited to, cocoa butter, beeswax and polyethylene glycols.

The pharmaceutical compositions of this invention may be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other solubilizing or dispersing agents known in the art.

Topical administration of the pharmaceutical compositions of this invention is especially useful when the desired treatment involves areas or organs readily accessible by topical application. For application topically to the skin, the pharmaceutical composition should be formulated with a suitable ointment containing the active components suspended or dissolved in a carrier. Carriers for topical administration of the compounds of this invention include, but are not limited to, mineral oil, liquid petroleum, white petroleum, propylene glycol, polyoxyethylene compound, emulsifying wax and water. Alternatively, the pharmaceutical composition can be formulated with a suitable lotion or cream containing the active compound suspended or dissolved in a carrier. Suitable carriers include, but are not limited to, mineral oil, sorbitan monostearate, polysorbate 60, cetyl esters wax, cetearyl alcohol, 2-octyldodecanol, benzyl alcohol and water. The pharmaceutical compositions of this invention may also be topically applied to the lower intestinal tract by rectal suppository formulation or in a suitable enema formulation. Topically-transdermal patches and iontophoretic administration are also included in this invention.

In the composition of the present application, ensartinib or a salt thereof of the present application is present in an pharmaceutically effective dose. The interrelationship of dosages for animals and humans (based on milligrams per meter squared of body surface) is described in Freireich et al., (1966) Cancer Chemother Rep 50: 219. Body surface area may be approximately determined from height and weight of the patient. See, e.g., Scientific Tables, Geigy Pharmaceuticals, Ardley, N. Y, 1970, 537. An effective amount of ensartinib or the salt thereof can range from about 5 mg/day to about 500 mg/day, more preferably 10 mg/day to about 400 mg/day, more preferably 25 mg/ day to about 250 mg/day. In some embodiments, the effective amount of ensartinib or the salt thereof can be 10 mg/day, 20 mg/day, 25 mg/day, 30 mg/day, 50 mg/day, 75 mg/day, 90 mg/ day, 100 mg/day, 120 mg/day, 150 mg/day, 180 mg/day, 200 mg/day, 225 mg/day, 250 mg/day, 300 mg/day, 350 mg/day, 400 mg/day etc. Effective doses will also vary, as recognized by those skilled in the art, depending on the diseases treated, the severity of the disease, the route of administration, the sex, age and general health condition of the patient, excipient usage, the possibility of co-usage with other therapeutic treatments such as use of other agents and the judgment of the treating physician.

The present application thirdly provides a method of treating diseases carrying MET exon 14 skipping mutation, which involves administering a therapeutically effective amount of ensartinib or a salt thereof, or a composition comprising ensartinib or a salt thereof to a subject in need, wherein, ensartinib has the structure shown in Formula I:

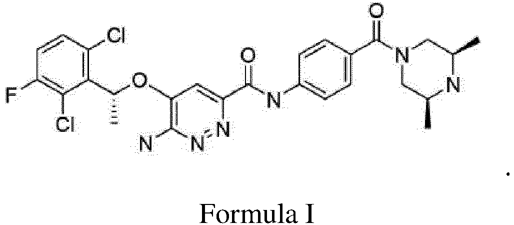

In some embodiments, an effective amount of ensartinib or a salt thereof, or a composition comprising ensartinib or a salt thereof, is administered to a subject in need by oral, rectal, nasal, topical, vaginal or parenteral administration.

In some embodiments, such effective amount is based on ensartinib or its salt, and the dose is about 5 mg/day to 500 mg/day, preferably 10 mg/day to 400 mg/day, more preferably 25 mg/day to 250 mg/day, and more preferably 225 mg/day.

The present application fourthly provides ensartinib or a salt thereof, or a composition containing ensartinib or a salt thereof for treating diseases carrying MET exon 14 skipping mutations, wherein, ensartinib has the structure shown in Formula I:

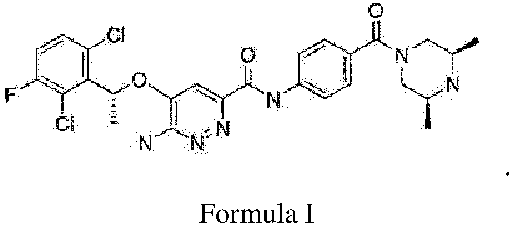

### The following abbreviations are used in the examples:

ADP: adenosine diphosphate;
ATP: adenosine triphosphate;
HPMC: hydroxypropyl methylcellulose;
DMSO: dimethyl sulfoxide;
FBS: fetal bovine serum;
PBS: phosphate buffered saline solution;
NSCLC: non-small cell lung cancer;
CR: complete response;
PR: partial response;
SD: stable disease;
DCR: disease control rate;
ORR: objective response rate;
CTG reagent: CellTiter-Glo reagent;
TGI(%): tumor growth inhibition rate.

### Example 1 Kinase inhibitory activity assay

The effects of Ensartinib on inhibition of MET exon 14 deletion (i.e. MET ex14Del) kinase were tested using ADP-Glo Kinase Assay Kit.

### Material:

MET(ex14Del) kinase(Sinalchem, M52-12PG);
ADP-Glo Kinase Assay Kit(Promega, V9102);

The experiment was conducted in a 384-well plate, the test compound ensartinib hydrochloride was initially prepared at a concentration of 300 µM. And then serially diluted 9 times with DMSO at a volume ratio of 1:3 to make 10 concentrations of 100× compound working solution, the diluted compound solution was added to a 384-well plate at a volume of 0.1 µL/well. 5 µL enzyme working solution was added to the 384-well plate, centrifuged at 1000 rpm for 1 minute, incubated 15 minutes at 25°C. 5 µL substrate working solution was added to each well to make 10 concentrations of 1× compound working solution by 3-fold serial dilutions, in which the maximum concentration of the compound solution is 3000nM, the reaction was triggered and incubated 60 minutes at 25°C. 10 µL ADP-Glo agent(Promega, V9102)was added to each well, centrifuged at 1000 rpm for 1 minute, incubated 15 minutes at 25°C, then 20 *µ*L kinase detection reagent was added to each well, centrifuged at 1000 rpm for 1 minute. RLU (relative luminescence units) was read by Envision Multimode Plate Reader, the value of RLU was used to characterize the degree of reaction between enzyme and substrate, IC₅₀ values were calculated by XLfit 5.3.1 nonlinear regression equation.

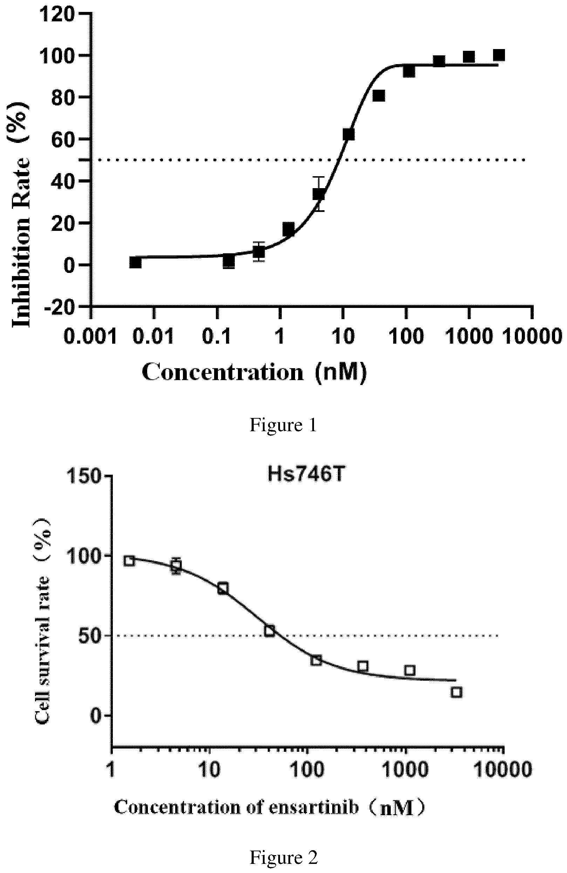

The relationship curve between MET exon 14 skipping mutation kinase inhibition rate and the concentration of ensartinib is shown in Figure 1, IC₅₀ (half inhibitory concentration) of Ensartinib in this application for MET (ex14Del) kinase is 7.6nM, it shows that ensartinib has excellent inhibitory effect on MET exon 14 skipping mutant kinase.

### Example 2 Cell proliferation inhibitory activity

CellTiter-Glo^{™} viable cells detection kit was used to evaluate inhibition effect of Ensartinib on human tumor cell line Hs746T.

### Material and cell line:

Human gastric cancer cell line Hs746T (harbor an MET exon 14 skipping mutation) provided by ATCC (American Type Cultural Collection), and then cultured in DMEM medium containing 10% FBS;
DMEM High glucose medium(Gibco, 11995-065);
Fetal bovine serum (FBS) (Gibco Cat#10099-141);
CellTiter-Glo^{™} Luminescent Cell Viability Assay Kit(Promega, Cat#G7573);
Ensartinib hydrochloride (Betta Phamaceuticals, Z170401T), diluted with DMSO to make a stock solution at a concentration of 10mM.

### Method:

1. Cell culture and plating
   1). Cells in logarithmic growth phase were harvested and counted using cell counter. Trypan blue exclusion test was used to measure the cell viability to make sure the cell viability is at least 90%.
   2). Adjust cell concentration, 180 µL cell suspension was added to each well of the 96-well plate at a density of 1200 cells/well.
   3).The 96-well plate was incubated overnight under 5%CO₂ and 95% humidity conditions at 37°C.
2. T0 Plate Reading
   1). 20 µL PBS was added to each well of the seeded 96-well plate.
   2). CTG agent was melted and the cell plate was equilibrated to room temperature for 30 minutes.
   3). 100 µL CTG solution was added to each well.
   4). The plate was placed on an orbital shaker for 5 minutes to lyse the cells.
   5). The plate was placed at room temperature for 20 minutes to stabilize the luminescent signal.
   6). T0 luminescence value (Lum) was recorded.
3. Drug dilution and dosing
   20 µL drug solution was added to each well of the seeded 96-well plate, concentration of Ensartinib is 10000 nM, 3333 nM, 1111 nM, 370 nM, 124 nM, 41 nM, 14 nM, 4.6 nM and 1.5 nM respectively: three wells were set for each drug concentration, and the final concentration of DMSO was 0.1%.
   3). The 96-well plate containing Ensartinib was incubated 168 hours under 5%CO₂ and 95% humidity conditions at 37°C, and then analyzed using CTG
4. Endpoint plate reading
   1). CTG agent was melted and the cell plate was equilibrated to room temperature for 30 minutes.
   2). 100 µL CTG solution was added to each well.
   3). The plate was placed on an orbital shaker for 5 minutes to lyse the cells.
   4). The plate was placed at room temperature for 20 minutes to stabilize the luminescent signal.
   5). Endpoint luminescence value (Lum) was recorded.

### Statistical analysis and result:

The data were analyzed using GraphPad Prism 7.0, in order to calculate IC₅₀, a dose-response curve was fitted using nonlinear regression model with a sigmoidal dose response. The surviving rate (%) = (Lumtest compound-Lummedium control)/(Lumcell control-Lummedium control)×100%.

The relationship curve between Hs746T cell survival rate and Ensartinib concentration curve is shown in Figure 2, Ensartinib of the present application inhibited the proliferation of Hs746T cells dose-dependently, IC₅₀ is 31 nM, it shows that Ensartinib has a significant inhibitory effect on human gastric cancer cells carrying MET exon 14 skipping mutations.

### Example 3 In Vivo Antitumor Activity Assays

### Experimental animals and Material;

BALB/c nude mice provided by Shanghai Sipur-Bika Experimental Animal Co., Ltd: SPF grade animals, 6-8 weeks old, weighing 18-23 grams; gender: female; number: 18 mice in the group.
Antibiotic(penicillin, streptomycin): (Gibco, 15240-062)_{∘}
Administration in solvent control group: Aqueous solution containing 0.5% HPMC and 0.4% Tween 80.

### Method:

### 1. Modeling

In order to prepare a Hs746T Xenograft Model, BALB/c Nude mice were inoculated subcutaneously with Hs746T cells (harbor an MET exon 14 skipping mutation, provided by ATCC (American Type Cultural Collection)).The animals were divided into control group and Ensartinib hydrochloride group (Ensartinib, 30 mg/kg), 6 animals per group. Test compounds were administered intragastrically starting from group date, twice a day for 18 days. Safety evaluation was performed based on animal weight changes and death, and efficacy evaluation was performed based on relative tumor inhibition rate (TGI%).

### 2. Cell culture

Human gastric cancer Hs746T cells were cultured single layer in vitro using DMEM medium containing 10 % FBS and 1% antibiotic solution, and incubated in a 5% CO₂, 37°C incubator. The cells were subcultured traditionally twice a week. The cells were harvested when the confluence reached 80%-90% and the number of cells reaches the required level, and then the cells were counted and seeded. 0.1 mL (2×10⁶) Hs746T cells were inoculated subcutaneously on the right back, and Ensartinib would be administrated when the tumor size reach about 128 mm³.

### 3. Tumor measurement and experimental indicators

Tumor diameters were measured twice a week with vernier calipers. The formula for calculating tumor volume was: V=0.5a×b2, where a and b are the long and short diameters of the tumor, respectively.

After the experiment, the tumor weight will be measured and TGI (%) will be calculated to reflect the rate of tumor growth inhibition. Calculation of TGI(%):TGI (%)=[1-(Average tumor volume at the end of administration in a certain treatment group-Average tumor volume at the beginning of administration in this treatment group)/(Average tumor volume at the end of treatment in the solvent control group-Average tumor volume at the beginning of treatment in the solvent control group)]×100%.

### Statistical analysis and result:

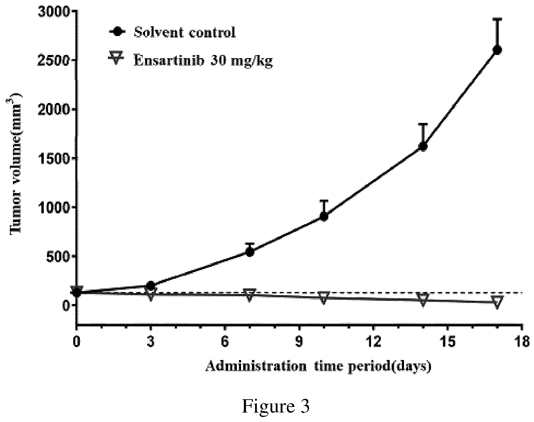

The average values of tumor volume at each time point for each group are shown in Table 1, and the tumor growth curve are shown in Figure 3. The results show that when tumor-bearing mice are given ensartinib hydrochloride, the growth of tumor was inhibited significantly and obvious tumor regression was observed. Ensartinib of the present invention has obvious effects on antitumor activity.

**Table 1 Tumor volume at different time points in each group**

| Days | Volume(mm³) | |
|---|---|---|
| | Solvent control | Ensartinib 30mg/kg |
| 0 | 128 ± 10 | 128 ± 10 |
| 3 | 199 ± 25 | 109 ± 8 |
| 7 | 546 ± 82 | 101 ± 8 |
| 10 | 908 ± 154 | 74 ± 9 |
| 14 | 1621 ± 228 | 49 ± 8 |
| 17 | 2605 ± 313 | 29 ± 15 |

Based on the above embodiments, Ensartinib of the present invention can significantly inhibit MET(ex14Del) kinase activity and proliferation of cells carrying MET exon 14 skipping mutation in vitro, and shows great anti-tumor activity in vivo, therefore it can be used to treat diseases carrying MET exon 14 skipping mutation, in further, Ensartinib or salts thereof and compositions containing Ensartinib or salts thereof can be used to treat diseases carrying MET exon 14 skipping mutation.

### Example 4 Clinical Study

Inclusion Criteria: Male or female adults, aged > 18 years; Patients had newly diagnosed histologically/cytologically confirmed stage IIIB/IV. NSCLC; Patients carrying a MET exon 14 skipping mutation; EGFR/ALK wild type; at least one measurable lesion.

Intervention: Ensartinib hydrochloride 225 mg was orally taken once a day until diseases progression or intolerable toxicity occurs.

Result: Among 17 patients, 1 case of CR, 11 cases of PR, and 4 cases of SD occurred. DCR reached 94% (16/17), ORR reached 71% (12/17); the average tumor shrinkage rate reached -37.7%. The above results show that Ensartinib has significant efficacy in diseases harboring MET exon 14 skipping mutations.

The above descriptions are only preferred embodiments of the present invention, and are not intended to limit the present invention, any modifications, equivalent substitutions, improvements, etc. made within the spirit and principles of the present invention shall be included in the protection scope of the present invention.

## Claims

1. Use of ensartinib or a salt thereof, or a composition comprising ensartinib or a salt thereof, in the treatment of diseases carrying MET exon 14 skipping mutation, wherein, ensartinib has the structure shown in Formula I:

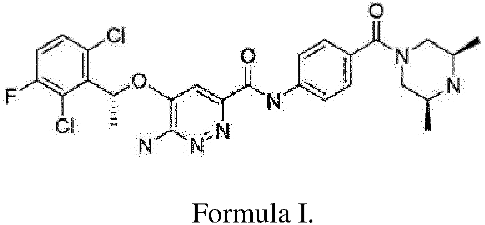

2. Use of ensartinib or a salt thereof, or a composition comprising ensartinib or a salt thereof, in the preparation of a medicament for treating diseases carrying MET exon 14 skipping mutation, wherein, ensartinib has the structure shown in Formula I:

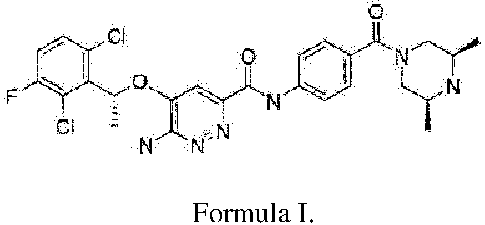

3. The use as defined in claim 1 or 2, wherein, the diseases carrying MET exon 14 skipping mutation include at least one of the cancers carrying MET exon 14 skipping mutation, wherein the cancers are selected from the group consisting of lung cancer, colon cancer, breast cancer, prostate cancer, liver cancer, pancreatic cancer, brain cancer, kidney cancer, ovarian cancer, stomach cancer, skin cancer, bone cancer, glioma, papillary renal cell carcinoma, and head and neck squamous cell carcinoma.

4. The use as defined in claim 1 or 2, wherein, the ensartinib or the salt thereof, or the composition comprising ensartinib or the salt thereof, is administered to a subject in need by oral, rectal, nasal, topical, vaginal or parenteral administration.

5. The use as defined in claim 1 or 2, wherein, the ensartinib or the salt thereof, or the composition comprising ensartinib or the salt thereof, is administered to a subject in need, at a dose of 5 mg/day to 500 mg/day in terms of ensartinib, preferably 10 mg/day to 400 mg/day, more preferably 25 mg/day to 250 mg/day.

6. The use as defined in any one of claims 1-5, wherein, the salt of ensartinib is one of hydrochloride, phosphate, formate, acetate, fumarate, oxalate, maleate, and citrate, preferably hydrochloride.

7. A method of treating diseases carrying MET exon 14 skipping mutation, comprising administering to a subject in need a therapeutically effective amount of ensartinib or a salt thereof, or a composition comprising ensartinib or a salt thereof, wherein, ensartinib has the structure shown in Formula I:

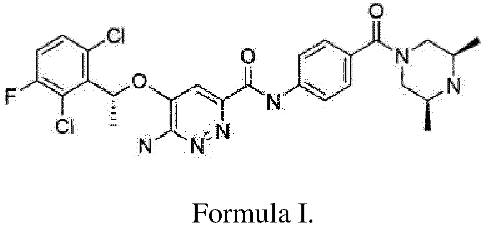

8. The method as defined in claim 7, wherein, administration is oral, rectal, nasal, topical, vaginal or parenteral.

9. The method as defined in claim 7, wherein, the effective amount is 5 mg/day to 500 mg/day in terms of ensartinib, preferably 10 mg/day to 400 mg/day, and more preferably 25 mg/day to 250 mg/day.

10. The method as defined in any one of claims 7-9, wherein, the salt of ensartinib is one of hydrochloride, phosphate, formate, acetate, fumarate, oxalate, maleate, and citrate, preferably hydrochloride.
